# EUROPEAN PATENT APPLICATION

(11) **EP 1 175 913 A1**
(43) Date of publication of application: **30.01.2002**
(21) Application number: 01118020.5
(22) Date of filing: 25.07.2001
(51) Int. Cl.: A61K 47/44, A61K 31/192, A61P 19/02, A61P 29/00

(54) **Intramuscular pharmaceutical composition comprising dexibuprofen and uses thereof**

(30) Priority: 28.07.2000 IT MI001741
(71) Applicant: SPA SOCIETA' PRODOTTI ANTIBIOTICI S.p.A., I-20143 Milan (IT)
(72) Inventor: Bruzzese, Tiberio, 20143 Milano (MI) (IT); Mozzi, Giovanni, 20143 Milano (MI) (IT); Bonabello, Angelo, 20143 Milano (MI) (IT)
(74) Representative: Mancini, Vincenzo, Dr.

(57) **Abstract**

The invention concerns an intramuscular pharmaceutical composition comprising the arylalkanoic nonsteroidal antiinflammatory agent (NSAID) dexibuprofen and/or a pharmaceutically acceptable salt thereof and at least one vegetable oil, affording high local tolerance. Its use for the preparation of a medicament to be administered by intramuscular route, for the treatment of disorders of the musculo-skeletal system and of pain is also described.

## Description

The invention concerns an intramuscular pharmaceutical composition comprising dexibuprofen and uses thereof; particularly, the invention relates to a composition comprising the arylalkanoic nonsteroidal anti-inflammatory agent (NSAID) dexibuprofen and/or a pharmaceutically acceptable salt thereof and at least one vegetable oil, and its use in the preparation of medicaments, to be administered by intramuscular route, for the treatment of disorders of the musculoskeletal system and of pain.

Dexibuprofen, namely S-(+)-ibuprofen is a pharmaceutical substance widely known and used in therapy. In addition to its anti-inflammatory action, it also shows an analgesic and antipyretic effect. It is therefore widely used in the treatment of musculoskeletal disorders such as ankylosing spondylitis, osteoarthritis, rheumatoid arthritis, bursitis, tendinitis, sprains, muscular strains, acute gout as well as in the relief of pain of whatever origin such as renal colic, dysmenorrhea, hemicrania, postoperative pain.

Administration of dexibuprofen and, generally of the other NSAIDs, may occur by oral, rectal, intramuscular and intravenous route.

The oral route, that would prove to be the simplest and easiest for the patient, is in practice characterised by the enhancement of the gastrointestinal side effects, peculiar of all the NSAIDs, such as gastric pain, nausea, vomit, gastrointestinal bleeding, peptic ulcer.

The rectal route is slowly becoming obsolescent in that disagreeable to many patients.

The intravenous route, problem-free in hospital, is not easily used at home.

The intramuscular route, affording unquestionable advantages for its promptness of action and reduced gastro-damaging effect, in comparison with the oral form, is however characterised by scarce local tolerance as shown in laboratory animals presenting hyperaemia, haemorrhages and hematomas at the site of injection.

The vehicle normally used in the formulation of intramuscular preparations of these products is mostly based on water or hydro-glycolic solutions, wherein the active ingredient is normally dissolved in the saline form or also suspended as such, to give depot preparations while, for the intravenous administration, solvents constituted by emulsions of oils in water are also adopted.

To provide some injectable NSAIDs, such as D-2-(6-methoxy)-2-naphtyl propionic acid (i.e. naproxen), in depot form, US 4,614,741 discloses suspending such NSAIDs (10-70% by weight) in 10-60% by weight suspending/dissolving agents, among which vegetable oils, such as almond oil. A depot effect reaching 2-4 days is reported.

US 5,554,650 discloses an antiphlogistic, analgesic and antipyretic parenteral preparation comprising diclofenac and/or its pharmaceutically acceptable salts, a surfactant like polyoxyethylenated oils (5-45%), a cosurfactant like an alcohol (2-15%), a watery buffer (about 50%) (and optionally an oil, 1-15%), giving a oil-in-water like microemulsion, affording reduced side effects upon parenteral administration, such as reduced pain at the injection site and more sustained release to achieve long-lasting therapeutic effects.

US 5,651,991 discloses a fatty emulsion of particles useful to improve drug (including NSAIDs) delivery upon parenteral administration, including a core constituted by lipids (30-85% w/w) and a surface layer covering said core consisting of a mixture of some lipids (among which some vegetable oils such as soybean, cotton seed, linseed, sesame, corn and peanut oils) and phospholipids or other surfactants (15-70% w/w). The technical problem which is said to be solved by the patented emulsions is basically to ameliorate the delivery of drugs to target tissues.

To overcome the gastroenteric trouble caused by the continuous administration of large amounts of flurbiprofen, US 4,613,505 describes a long-lasting intravenous emulsion comprising a phospholipid emulsifier and particles of soybean oil which contain an effective amount of an ester of flurbiprofen, in an aqueous medium.

WO 99/27906 discloses long-acting injectable formulations comprising among other drugs some NSAIDs (1-10% w/v), hydrogenated castor oil (0.3-5% w/v) and a hydrophobic carrier comprising 1) (30-45% w/v) triacetin, benzyl benzoate or ethyl oleate or a combination thereof and 2) (55-70% w/v) acylated monoglycerides, propyldicaprilates/dicaprates, caprylic/capric acid triglycerides or a combination thereof.

EP-A-429,248 describes a long lasting emulsified composition comprising 1) a lipid-soluble drug (among them NSAIDs) and a lipid; 2) a sugar, a sugar alcohol or a mixture thereof; 3) water; 4) a water-soluble non-ionic surfactant or a mixture thereof with a phospholipid in peculiar ratios.

DE 4,322,826 discloses a pharmaceutical composition, for oral administration, comprising a hardly water-soluble active principle (for instance ibuprofen and dexibuprofen) and a carrier, wherein the carrier includes 1) polygliceryl and/or sorbitan esters, 2) a pharmaceutically suitable oil containing a triglyceride as lipophilic component and 3) non-ionic surfactants.

The applicant has realised that there is still the need to improve the local tolerability of pharmaceutical compositions comprising NSAIDs intramuscularly administered and that the solutions above illustrated do not still solve this technical problem. Further and evidently, when a prompt effect of the drug is desired, such known pharmaceutical compositions reveal to be unsuitable.

A pharmaceutical composition has now been found, comprising dexibuprofen and/or at least one of the pharmaceutically acceptable salts thereof and at least one vegetable oil, which surprisingly overcomes the above problems.

In fact, according to a first aspect, the invention concerns an intramuscular pharmaceutical composition comprising a solution of dexibuprofen and/or at least one of the pharmaceutically acceptable salts thereof and at least one vegetable oil. It is preferred that the solution be anhydrous.

The pharmaceutically acceptable salts are preferably selected among the alkaline salts, the lysine and arginine salts of dexibuprofen.

It was surprisingly found that the composition of the invention can be administered by intramuscular route, without the onset of the phenomena characterising scarce local tolerance of dexibuprofen itself, as well as of the other arylalkanoic NSAIDs, in mostly water or hydroglycolic solutions. The composition of the invention showed unexpectedly a high local tolerance.

Surprisingly, such strong improvement of the local tolerability was not obtained with oily solutions of other NSAIDs.

It was also unexpectedly observed that the absence of local intolerance of the composition of the invention was disjoined by any reduction of pharmacological activity: for instance, the analgesic activity of dexibuprofen, controlled with the tail flick test in mice (see Example 6 below), remained almost unchanged, with the onset of action slightly more prompt, but with overall potency substantially similar and duration of action lasting not more than a few hours (2-3 h) in both cases. It means that bioavailability and half-life of the drug is almost unchanged when injected in watery or in oily solutions. Oily solutions seem however to induce a more prompt effect.

In conclusion, it is definitely demonstrated that the administration of the composition of the invention forestalls the tissue damages connected with intramuscular injection of dexibuprofen and other arylalkanoic NSAIDs in watery solutions while retaining full activity. It was also found that such unexpected effect cannot be obtained with oily solutions of other NSAIDs. This obviates admission of the patient to hospital, as is the case with intravenous route, with therapeutic action quicker than that of the other administration routes (oral and rectal ones) and without the pronounced gastro-damaging effects of the oral form.

The concentration of dexibuprofen in the composition of the invention varies, preferably, from 20 mg/ml to 100 mg/ml, in particular from 25 mg/ml to 80 mg/ml (i.e. the concentration is less than or equal to 10% w/v). Total quantities of 100-200 mg, or even up to 300-400 mg of dexibuprofen, can be however easily administered, as required.

The vegetable oil is selected among, preferably, peanut oil, almond oil, olive oil, castor oil, sesame oil, soybean oil; refined almond oil is particularly preferred.

According to another preferred aspect, the composition of the invention also comprises at least a pharmaceutically acceptable excipient, easily selected by the skilled man, among those ordinarily used; for example polysorbate 80, Cremophor® , EL, lecithin, triacetin, benzyl alcohol.

According to a further aspect, the invention concerns the use of the above defined composition for the preparation of a medicament, to be administered by intramuscular route, for the treatment of the disorders of the musculoskeletal system such as, for instance, ankylosing spondylitis, osteoarthritis, rheumatoid arthritis, bursitis, tendinitis, sprains, muscular strains, acute gout.

According to a further aspect, the invention concerns the use of the above defined composition in the preparation of an analgesic medicament, to be administered by intramuscular route, for the treatment of pain of whichever origin, deriving, for instance, from renal or hepatic colics, dysmenorrhea, hemicrania, postoperative pain, dental extraction and/or dental surgery and cancer pain.

In all such cases, the highest Therapeutic Index, i.e. the ratio of the maximum dose not giving local intolerance at the injection site to the minimum dose already giving therapeutic effects, has been achieved by administering the composition of the invention.

The composition of the invention can be prepared by dissolving or vehiculating dexibuprofen and/or the pharmaceutically acceptable salts thereof, preferably in a concentration ranging from 20 mg/ml to 100 mg/ml, in particular 25-80 mg/ml, in at least one vegetable oil. In the composition of the invention, unlike water solutions, dexibuprofen is preferably used as an acid.

The following examples illustrate the invention without limiting it.

### Example 1

Formulation of an ampoule for the intramuscular administration of dexibuprofen:

| | |
|---|---|
| Dexibuprofen | 200 mg |
| Refined almond oil, q.s. to | 2 ml |

To prepare the composition according to the invention, dissolve the active ingredient in the oil, dilute to the final volume, under sterile conditions filter the resulting solution through a 0.22µ membrane and, still under sterile conditions, distribute into neutral glass ampoules.

### Example 2

Formulation of an ampoule for the intramuscular administration of dexibuprofen:

| | |
|---|---|
| Dexibuprofen | 200 mg |
| Refined almond oil, q.s. to | 2.5 ml |

To prepare the composition according to the invention, dissolve the active ingredient in the oil, dilute to the final volume, under sterile conditions filter the resulting solution through a 0.22µ membrane and, still under sterile conditions, distribute into neutral glass ampoules.

### Example 3

Formulation of an ampoule for the intramuscular administration of dexibuprofen:

| | |
|---|---|
| Dexibuprofen | 100 mg |
| Refined almond oil, q.s. to | 2.0 ml |

To prepare the composition according to the invention, dissolve the active ingredient in the oil, dilute to the final volume, under sterile conditions filter the resulting solution through a 0.22µ membrane and, still under sterile conditions, distribute into neutral glass ampoules.

### Example 4

The tolerance of the composition of the invention was evaluated in rats following intramuscular administration.

Male SD rats, weighing 250-270 g, were used in the test.

The test products (at the concentration of 100 mg/ml) were injected into the thigh muscle through a 0.45 x 13 mm needle, in the volume of 1 ml/kg. 24 hours thereafter the animals were sacrificed under ether anaesthesia and the treated muscles were sectioned to evidence the site of injection.

Any possible local damage was evaluated through the following rating scale attributed to each animal:
0 = Normal and/or mild hyperaemia
1 = Hyperaemia with haemorrhagic area of < 1 cm
2 = Hyperaemia with haemorrhagic area of > 1 cm
3 = Hematoma and haemorrhagic area of > 1 cm
4 = Diffused hematoma of > 2 cm

The compositions A, B, C and D considered for the test were formulated as follows:

| Composition | A* | B* | C* | D |
|---|---|---|---|---|
| Dexibuprofen | 300 mg | 300 mg | 300 mg | 300 mg |
| Benzyl alcohol | - | 60 mg | 60 mg | - |
| Propylene glycol | - | - | 900 mg | - |
| 1/N NaOH | q.s. to | q.s. to | q.s. to | - |
| | pH 7.4 | H 7.4 | pH 7.4 | |
| Water | q.s. to 3 ml | q.s. to 3 ml | q.s. to 3 ml | - |
| Refined almond oil | - | - | - | q.s. to 3 ml |

| | | | | |
|---|---|---|---|---|
| * - Comparison | | | | |

The results obtained are reported in the following table:

| Local tolerance after intramuscular administration of 100 mg/kg | | | | |
|---|---|---|---|---|
| | Composition | | | |
| | A* | B* | C* | D |
| No. of animals | 15 | 9 | 10 | 8 |
| Mean index | 1.5 | 1 | 2.6 | 0 |
| Standard deviation | 1.30 | 1.12 | 1.07 | 0 |
| Significance vs D | P<0.005 | P<0.05 | P<0.001 | - |

| | | | | |
|---|---|---|---|---|
| * - Comparison | | | | |

It can be seen that the tolerance of the composition of the invention is definitely and significantly (Student's t test) better than the tolerance of the reference compositions.

### Example 5

The tolerance of three NSAIDs compositions (A-C) was evaluated and compared with the composition of the invention (D) in male rats weighing 250-275 g, following intramuscular administration.

| Composition | A* Ibuprofen | B* Naproxen | C* Diclofenac | D Dexibuprofen |
|---|---|---|---|---|
| NSAID | 200 mg | 200 mg | 75 mg | 200 mg |
| Benzyl alcohol | 60 mg | - | - | - |
| Propylene glycol | 900 mg | - | - | - |
| 1/N NaOH | q.s. to | | | |
| | pH 7.4 | - | - | - |
| Water | q.s. to | | | |
| | 2.5 ml | - | - | - |
| Refined almond oil | - | q.s. to | q.s. to | q.s. to 2.5 |
| | | 2.5 ml | 2.5 ml | ml |

| | | | | |
|---|---|---|---|---|
| *-Comparison | | | | |

The solutions were injected into the thigh muscle through a 0.45 x 13 mm needle, in the volume of 1 ml/kg. 24 hours thereafter the animals were sacrificed under ether anaesthesia and the treated muscles were sectioned to evidence the site of injection.

Any possible local damage was evaluated through the same rating scale of Example 4.

The results obtained are reported in the following table:

| Local tolerance after intramuscular administration of 80 mg/kg | | | | |
|---|---|---|---|---|
| Composition | A* | B* | C* | D |
| No. of animals | 10 | 10 | 10 | 10 |
| Mean index | 2.3 | 2.1 | 2.9 | 0 |
| Standard deviation | 1.12 | 0.72 | 1.03 | 0 |
| Significance vs D | p<0.001 | p<0.001 | p<0.001 | - |

| | | | | |
|---|---|---|---|---|
| *-Comparison | | | | |

The local tolerance of the composition of the invention D, following i.m. administration at 80 mg/kg, is definitely and significantly (Student's t test) better than the tolerance of the reference compositions A-C.

### Example 6

The analgesic efficacy of two different compositions, administered by intramuscular route, of dexibuprofen, one in an aqueous vehicle and the other in an oily vehicle, was tested in mice through the Tail-flick test described by Dewey W.L., Harris L.S., Howes S.F., Nuite J.A., in J. Pharmacol. Exp. Ther., 175 (1970), 435-442.

The test entails determining the delay in the response to pain produced by heat (light ray on tail) after treatment with the test substance. By administering varying doses of the substance and evaluating the different responses it was possible to determine the effective dose, ED₅₀, i.e. the dose inducing an increase, by 50%, of the baseline response time.

The compositions examined were formulated as follows:

| | A (comparison) | B |
|---|---|---|
| Dexibuprofen | 100 mg | 100 mg |
| 1/N NaOH | q.s. to pH 7.4 | - |
| Water | q.s. to 2 ml | - |
| Refined almond oil | - | q.s. to 2 ml |

The compositions were administered at 3 different dosages (100, 50 and 25 mg/kg) to each of 10 animals.

The most diluted dosages (50 and 25 mg/kg) were obtained from compositions A and B, by suitable dilution with water for injections and refined almond oil respectively.

ED₅₀ was determined 30, 60 and 120 minutes after administration.

The results are reported in the following table:

| Analgesic activity following intramuscular administration (Tail flick) | | |
|---|---|---|
| | Composition A (comparison) | Composition B |
| Time from administration (minutes) | ED₅₀ (confidence limits) | ED₅₀ (confidence limits) |
| 30 | 87.3 (76.5 -98.8) | 50.7 (31.6 - 81.2) |
| 60 | 65.4 (43.9 - 83.3) | 64.6 (56.2 - 74.9) |
| 120 | 950 (512.3 -1820.2) | 1088.8 (541.2 - 2717.8) |

It can be seen that the efficacy of the two compositions was alike. What is more, after 30 minutes, composition B presented an ED₅₀ lower (that is, better efficacy) than that of reference composition A, this showing the higher prompt effect of the composition of the invention.

## Claims

1. An intramuscular pharmaceutical composition comprising a solution of dexibuprofen and/or at least one of the pharmaceutically acceptable salts thereof and at least one vegetable oil.

2. A composition according to claim 1, wherein the pharmaceutically acceptable salts are selected among the alkaline salts, the lysine and arginine salts.

3. A composition according to claim 1 or 2, wherein the concentration of dexibuprofen ranges from 20 mg/ml to 100 mg/ml.

4. A composition according to any of the foregoing claims, wherein the concentration of dexibuprofen ranges from 25 mg/kg to 80 mg/kg.

5. A composition according to any of the foregoing claims, wherein the solution is anhydrous.

6. A composition according to any of the foregoing claims, wherein the vegetable oil is selected from peanut oil, almond oil, olive oil, castor oil, sesame oil, soybean oil.

7. A composition according to any of the foregoing claims, wherein the vegetable oil is refined almond oil.

8. A composition according to any of the foregoing claims, comprising at least a pharmaceutically acceptable excipient.

9. Use of the composition according to any of the foregoing claims for the preparation of a medicament, to be administered by intramuscular route, for the treatment of the disorders of the musculo-skeletal system.

10. Use according to the previous claim, wherein the disorder of the musculo-skeletal system is ankylosing spondylitis, osteoarthritis, rheumatoid arthritis, bursitis, tendinitis, sprains, muscular strains, acute gout.

11. Use of the composition according to any of claims 1 to 8, for preparation of a medicament, to be administered by intramuscular route, for the treatment of pain conditions.

12. Use according to the previous claim, wherein pain derives from renal or hepatic colics, dysmenorrhea, hemicrania, postoperative pain, dental extraction and/or dental surgery and cancer pain.
